(19) Europäisches Patentamt
European Patent Office
Office européen des brevets

(11) **EP 2 387 579 B1**

(12) **FASCICULE DE BREVET EUROPEEN**

(45) Date de publication et mention
de la délivrance du brevet:
**12.03.2014 Bulletin 2014/11**

(51) Int Cl.:
**C07K 7/08** *(2006.01)*       **A61K 47/48** *(2006.01)*
**C07K 14/47** *(2006.01)*

(21) Numéro de dépôt: **10704386.1**

(22) Date de dépôt: **19.01.2010**

(86) Numéro de dépôt international:
**PCT/FR2010/000045**

(87) Numéro de publication internationale:
**WO 2010/081975 (22.07.2010 Gazette 2010/29)**

(54) **POLYPEPTIDES D'ADRESSAGE SPECIFIQUE A DES CELLULES-CIBLES D'OTX2**

POLYPEPTIDE FÜR DAS SPEZIFISCHE TARGETING GEGEN OTX2-TARGETZELLEN

POLYPEPTIDES FOR SPECIFIC TARGETING TO OTX2 TARGET CELLS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.01.2009 FR 0900217**

(43) Date de publication de la demande:
**23.11.2011 Bulletin 2011/47**

(73) Titulaires:
• **Centre National de la Recherche Scientifique
75016 Paris (FR)**
• **Ecole Normale Supérieure
75230 Paris Cedex 05 (FR)**

(72) Inventeurs:
• **PROCHIANTZ, Alain
F-75006 Paris (FR)**
• **DI NARDO, Ariel
F-91120 Palaiseau (FR)**
• **BEURDELEY, Marine
F-75014 Paris (FR)**

(74) Mandataire: **Vialle-Presles, Marie José
Cabinet ORES
36, rue de St Pétersbourg
75008 Paris (FR)**

(56) Documents cités:
**WO-A-2009/106767**

• **DATABASE UniProt [Online] 1 octobre 1993 (1993-10-01), "RecName: Full=Homeobox protein OTX2; AltName: Full=Orthodenticle homolog 2;" XP002546491 extrait de EBI accession no. UNIPROT:P32243 Database accession no. P32243 cité dans la demande**
• **RATH M.F. ET AL.: "Ontogenetic expression of the Otx2 and Crx homeobox genes in the retina of the rat" EXPERIMENTAL EYE RESEARCH, ACADEMIC PRESS LTD, LONDON, vol. 85, no. 1, 1 juillet 2007 (2007-07-01), pages 65-73, XP022153829 ISSN: 0014-4835**
• **PROCHIANTZ A.: "Processus morphologiques" 10 décembre 2007 (2007-12-10), pages 260-262, XP002576751 College de France Extrait de l'Internet: URL:http://www.college-de-france.fr/ media/ pub_tra/UPL2853_resume_cours_et_ travaux_07 _08.pdf> [extrait le 2010-04-08]**
• **HUANG Z JOSH ET AL: "Time to change: Retina sends a messenger to promote plasticity in visual cortex" NEURON, vol. 59, no. 3, août 2008 (2008-08), pages 355-358, XP002576801 ISSN: 0896-6273**
• **REBSAM ALEXANDRA ET AL: "Otx2's incredible journey" CELL, vol. 134, no. 3, août 2008 (2008-08), pages 386-387, XP002576802 ISSN: 0092-8674**
• **SUGIYAMA SAYAKA ET AL: "Experience-dependent transfer of Otx2 homeoprotein into the visual cortex activates postnatal plasticity" CELL, vol. 134, no. 3, août 2008 (2008-08), pages 508-520, XP002576803 ISSN: 0092-8674**

EP 2 387 579 B1

• SUGIYAMA SAYAKA ET AL: "From brain formation to plasticity: Insights on Otx2 homeoprotein" DEVELOPMENT GROWTH & DIFFERENTIATION, vol. 51, no. 3, avril 2009 (2009-04), pages 369-377, XP002576804 ISSN: 0012-1592

• BAAS D. ET AL.: "The subcellular localization of Otx2 is cell-type specific and developmentally regulated in the mouse retina" MOLECULAR BRAIN RESEARCH, ELSEVIER SCIENCE BV, AMSTERDAM, NL, vol. 78, no. 1-2, 31 mai 2000 (2000-05-31), pages 26-37, XP008108487 ISSN: 0169-328X

**Description**

La présente invention est relative à des polypeptides permettant l'adressage spécifique d'une molécule d'intérêt à des cellules-cibles de l'homéoprotéine Otx2 et notamment aux neurones ganglionnaires et aux neurones bipolaires rétiniens.

La rétine est le feuillet cellulaire tapissant le fond de l'oeil. Elle contient différents types de neurones dont le rôle est de capter l'énergie lumineuse et de la transformer en signal nerveux, ainsi que des cellules gliales.

Schématiquement, la rétine comprend trois couches principales de neurones : les neurones photorécepteurs (cônes et bâtonnets), les neurones bipolaires, et les neurones ganglionnaires ; d'autres neurones, les neurones amacrines et les neurones horizontaux, jouent un rôle régulateur. Les neurones photorécepteurs réagissent à la lumière, et le signal qu'ils génèrent est transmis par l'intermédiaire des neurones bipolaires, aux neurones ganglionnaires, dont les axones constituent les fibres nerveuses du nerf optique, assurant l'envoi de l'information au cerveau.

La dégénérescence des neurones rétiniens est impliquée dans diverses rétinopathies. Ainsi, la dégénérescence des neurones photorécepteurs est impliquée dans certaines pathologies, telles que la rétinite pigmentaire ou la dégénérescence maculaire. Dans d'autres pathologies, telles que le glaucome, ce sont principalement les neurones ganglionnaires qui sont atteints.

Otx2 (Orthodenticle homolog 2) est une homéoprotéine contenant un homéodomaine de type bicoïd (SIMEONE et al., Embo J, 12, 2735-47, 1993). Elle appartient à la famille d'homéoprotéines Otx, qui joue un rôle fondamental dans le développement du cerveau au cours de l'embryogenèse (ACAMPORA et al., Prog Neurobiol, 64, 69-95, 2001; SIMEONE et al., Curr Opin Genet Dev, 12, 409-15, 2002).

Lors de précédents travaux, l'équipe des Inventeurs a mis en évidence l'effet positif d'une homéoprotéine, Otx2 (Orthodenticle homolog 2) sur la survie des neurones ganglionnaires rétiniens. Ces résultats sont rapportés dans la Demande PCT WO/2009/106767.

Il a également été montré qu'Otx2 jouait un rôle dans le développement post-natal du cortex cérébral, et notamment dans sa plasticité. Otx2 qui est synthétisé au niveau de la rétine, est transporté jusqu'au cortex visuel, où il est capturé par les neurones à parvalbumine du cortex visuel, ce qui induit leur maturation, et entraine d'abord l'ouverture, puis quelque temps après, la fermeture de la période critique de plasticité (SUGIYAMA et al., Cell, 134, 508-20, 2008).

Une période critique de plasticité est une phase du développement post-natal représentée par un intervalle de temps de durée limitée (variable selon l'espèce animale, et selon la fonction sensorielle concernée) pendant lequel les stimuli sensoriels sont capables de modifier l'organisation fonctionnelle des zones corticales correspondantes.

En poursuivant leurs recherches, les Inventeurs ont découvert que dans la rétine, Otx2 se fixait spécifiquement aux neurones ganglionnaires et aux neurones bipolaires, et ont identifié la région d'Otx2 responsable de cette fixation. Ils ont en outre montré que cette même région était également responsable de l'adressage d'Otx2 vers les neurones à parvalbumine du cortex visuel, en interagissant avec le réseau périneuronal de protéoglycannes à chondroïtine sulfate qui enrobe ces neurones, et qu'elle permettait en entrant en compétition avec la protéine Otx2 endogène capturée par les neurones à parvalbumine, d'inhiber cette capture. Cette inhibition entraîne le retour de ces neurones à une état immature, permettant la réouverture de la phase critique de plasticité.

La région responsable de l'adressage d'Otx2 à ses cellules cibles est constituée par une séquence peptidique de 15 acides aminés. Les Inventeurs ont en outre constaté que ce polypeptide, isolé, possédait la même spécificité de fixation que la protéine Otx2 entière.

La présente invention a pour objet un polypeptide d'adressage cellulaire isolé défini par la séquence $B_1B_2X_1B_3B_4X_2B_5X_3X_4X_5B_6X_7X_8X_9$ (SEQ ID NO: 1), dans laquelle :

$B_1$, $B_2$, $B_3$, $B_4$, $B_5$ et $B_6$ représentent indépendamment l'arginine ou la lysine ;
$X_1$ et $X_8$ représentent indépendamment l'asparagine ou la glutamine ;
$X_2$ représente l'acide aspartique ou l'acide glutamique ;
$X_3$, $X_4$ et $X_6$ représentent indépendamment la thréonine ou la sérine ;
$X_5$ représente la phénylalanine, la tyrosine ou le tryptophane ;
$X_7$ représente l'alanine ou la glycine ;
$X_9$ représente la leucine, l'isoleucine ou la valine.

Ce polypeptide, en présence de cellules rétiniennes, se lie spécifiquement aux neurones ganglionnaires et aux neurones bipolaires.

Des modes de réalisation préférés de la présente invention sont des polypeptides dans lesquels :

- au moins un des acides aminés $B_1$, $B_3$, $B_4$, $B_5$ et $B_6$ est une arginine ; et/ou
- $B_2$ est une lysine ; et/ou
- au moins un des acides aminés $X_1$ et $X_8$ est une glutamine ; et/ou

- X₂ est un acide glutamique ; et/ou
- au moins un des acides aminés X₃ X₄ et X₆ est une thréonine ; et/ou
- X₅ est une phénylalanine ; et/ou
- X₇ est une alanine ; et/ou
- X₉ est une leucine.

Selon un mode de réalisation particulièrement préféré d'un polypeptide d'adressage cellulaire conforme à l'invention il est défini par la séquence suivante : RKQRRERTTFTRAQL (SEQ ID NO: 2).

Les acides aminés constituant un polypeptide conforme à l'invention peuvent être des acides aminés naturels, de la série L. On peut également remplacer tout ou partie de ces acides aminés par leurs isomères de la série D, afin d'augmenter la stabilité du polypeptide *in vivo.* Les acides aminés, de la série L ou de la série D, peuvent également, le cas échéant, être enchaînés selon une séquence inverse de la séquence SEQ ID NO: 1 ou de la séquence SEQ ID NO: 2 indiquées ci-dessus.

La présente invention a également pour objet l'utilisation d'un polypeptide d'adressage cellulaire conforme à l'invention pour permettre l'adressage spécifique d'un cargo d'intérêt à des cellules-cibles d'Otx2.

A titre d'exemples de cellules-cibles d'Otx2, on citera, outre les neurones ganglionnaires et les neurones bipolaires rétiniens déjà mentionnés ci-dessus, les neurones enrobés dans un réseau périneuronal de protéoglycannes à chondroïtine sulfate, ce qui inclut notamment des neurones exprimant la parvalbumine (neurones PV) localisés notamment dans le cortex visuel. D'autres cellules-cibles d'Otx2 sont des neurones du cerveau moyen, en particulier les neurones dopaminergiques de la substance noire et de l'aire tegmentale ventrale et leurs cibles synaptiques.

D'autres cellules-cibles d'Otx2 peuvent aisément être identifiées à l'aide d'un polypeptide d'adressage conforme à l'invention, par exemple en associant ledit polypeptide à un marqueur, en mettant en contact ledit polypeptide marqué avec un échantillon d'un tissu ou d'un organe à tester, et en détectant, dans ledit échantillon, la présence ou l'absence de cellules fixant ledit polypeptide, et en cas de présence de cellules fixant ledit polypeptide, leur localisation.

On désigne sous le terme général de : " cargo ", toute molécule ou complexe moléculaire, que l'on souhaite adresser à une cellule-cible.

Les cargos pouvant être transportés par des polypeptides d'adressage cellulaire conformes à l'invention peuvent être de nature très variée : il peut s'agir de molécules chimiques, de macromolécules, telles par exemple que des protéines ou des acides nucléiques, ou de particules telles que des liposomes, des nanoparticules, ou des particules virales ou pseudovirales. Il peut s'agir de marqueurs destinés à permettre de détecter et/ou de localiser des cellules-cibles d'Otx2 dans un tissu ou un organe, ou de principes actifs que l'on souhaite adresser spécifiquement à des cellules-cibles d'Otx2.

Si l'on souhaite obtenir non seulement l'adressage du cargo d'intérêt à la cellule-cible, mais également son entrée dans celle-ci, on peut avantageusement associer un polypeptide d'adressage cellulaire conforme à l'invention à un polypeptide transducteur.

Les polypeptides transducteurs sont des polypeptides comprenant une séquence dénommée « domaine de transduction » leur conférant la capacité de pénétrer à l'intérieur d'une cellule vivante, indépendamment de la présence de transporteurs ou de récepteurs spécifiques.

De très nombreux polypeptides transducteurs sont connus en eux-mêmes. A titre d'exemples non limitatifs, on citera : les pénétratines, qui sont des polypeptides dérivés de la troisième hélice d'un homéodomaine ; les polypeptides dérivés de la protéine Tat de HIVI, et en particulier du fragment 48-60 de ladite protéine ; les polyarginines ; les polypeptides dérivés de la protéine VP22 de HSV ; des polypeptides dérivés d'une séquence signal conjuguée à une séquence de localisation nucléaire ; les transportanes qui sont dérivés d'une fusion entre une portion d'un neuropeptide, la galanine, et un polypeptide du venin de guêpe.

La présente invention a également pour objet un polypeptide isolé contenant un polypeptide d'adressage cellulaire conforme à l'invention et un polypeptide transducteur.

Ce polypeptide peut être un fragment isolé d'Otx2 comprenant la totalité de l'homéodomaine, et les 2 acides aminés précédant immédiatement celui-ci. Ce fragment peut également être délété d'une partie de la séquence de l'homéodomaine pourvu que la séquence d'adressage conforme à l'invention et au moins la troisième hélice de l'homéodomaine soient conservées.

Il peut également s'agir d'un polypeptide chimérique associant un polypeptide d'adressage cellulaire conforme à l'invention avec un polypeptide transducteur hétérologue. Dans ce cadre, des polypeptides transducteurs préférés sont ceux de la famille des pénétratines. On peut ainsi associer un polypeptide d'adressage cellulaire conforme à l'invention avec un fragment d'homéodomaine d'une homéoprotéine autre qu'Otx2, comprenant au moins la troisième hélice dudit homéodomaine, ou bien avec des dérivés de pénétratine tels que ceux décrits par exemple dans les Demandes PCT WO 00/01417, ou WO 00/29427.

La présente invention a également pour objet des compositions comprenant un polypeptide d'adressage cellulaire conforme à l'invention, éventuellement associé avec un polypeptide transducteur, lié à un cargo.

La liaison entre le polypeptide d'adressage cellulaire conforme à l'invention et le cargo peut s'effectuer de différentes manières, connues en elles-mêmes, selon notamment la nature du cargo concerné, et les modalités d'utilisation envisagées. Généralement, le polypeptide d'adressage cellulaire (éventuellement fusionné avec un polypeptide transducteur) et le cargo seront associés de manière covalente, le cas échéant par l'intermédiaire d'un bras espaceur, par exemple un lieur peptidique. Ils peuvent également être associés de manière non-covalente, par l'intermédiaire d'interactions ioniques ou hydrophobes ; dans ce cas, le polypeptide d'adressage peut être lié à une molécule, capable de se lier de manière non-covalente au cargo. Cette molécule peut en particulier être un polypeptide transducteur tel qu'une pénétratine, capable de se lier par interactions hydrophobes à un cargo possédant un ou plusieurs domaines hydrophobes, comme décrit dans la Demande PCT WO 04/069279.

Selon un mode de réalisation particulier d'une composition conforme à l'invention, ladite composition se présente sous forme d'un polypeptide chimérique, comprenant un polypeptide d'adressage cellulaire conforme à l'invention, lié avec une ou plusieurs séquences polypeptidiques constituant le cargo, et éventuellement avec un polypeptide transducteur. L'ordre dans lequel sont disposés le polypeptide d'adressage cellulaire, le polypeptide transducteur, et les séquences polypeptidiques constituant le cargo n'est pas critique.

A titre d'exemples non-limitatifs de polypeptides chimériques conformes à l'invention on citera des polypeptides chimériques comprenant un polypeptide d'adressage cellulaire conforme à l'invention, un polypeptide transducteur, une ou plusieurs séquences de régulation de la transcription et/ou une ou plusieurs séquences de régulation de la traduction. Le terme polypeptide chimérique est utilisé ici dans son sens usuel, pour désigner des polypeptides associant des séquences d'origine différentes, ce qui exclut donc les protéines Otx2 natives.

De nombreuses séquences de régulation de la transcription ou de régulation de la traduction sont connues en elles-mêmes.

A titre d'exemples, on citera :

- des séquences d'activation de la transcription telles par exemple que le trans-activateur VP16 du virus HSV (herpes simplex virus) ;
- des séquences de répression de la transcription telles que celle d'Engrailed (correspondant par exemple aux acides aminés 1-298 dans la protéine Engrailed de *Drosophila melanogaster* (GenBank AAA65478).
- des séquences de régulation (notamment d'activation) de la traduction, telles que des sites de liaison à eIF4E qui ont par exemple été détectés dans de nombreuses homéoprotéines (pour revue cf. TOPISIROVIC & BORDEN, Histol. Histopathol., 20, 1275-1284, 2005), dont Otx2.

Les polypeptides chimériques conformes à l'invention peuvent être obtenus par différentes méthodes bien connues en elles-mêmes, notamment par synthèse peptidique, ou par les techniques classiques du génie génétique.

Des polypeptides chimériques conformes à l'invention comprenant un polypeptide d'adressage cellulaire, un polypeptide transducteur, une ou plusieurs séquences de régulation de la transcription et/ou une ou plusieurs séquences de régulation de la traduction sont utilisables dans les mêmes applications que la protéine Otx2 native, et notamment pour augmenter la survie des cellules cibles d'Otx2. Ils sont ainsi utilisables en particulier pour prévenir ou traiter la dégénérescence des neurones ganglionnaires et/ou des neurones bipolaires rétiniens, qui intervient notamment dans le glaucome, ainsi que dans diverses neuropathies optiques, génétiques ou vasculaires, par exemple la rétinite pigmentaire ou les lésions du nerf optique. Ils sont également utilisables dans le cadre du traitement de certaines maladies neurodégénératives (telles que par exemple la maladie d'Alzheimer, la sclérose en plaques ou la maladie de Parkinson). Généralement, ces polypeptides chimériques pourront être utilisés dans lesdites applications selon les mêmes modalités que celles décrites pour Otx2 dans la demande PCT WO/2009/106767.

Pour la mise en oeuvre de la présente invention, il suffit de mettre ledit polypeptide chimérique en contact avec les cellules cibles ; il pénètre en effet dans celles-ci grâce à la séquence d'internalisation fournie par le polypeptide transducteur. De manière préférée ladite mise en contact est effectuée à une concentration dudit polypeptide chimérique de 0,5 à 10 nM, avantageusement de 1 à 5 nM, et de manière particulièrement avantageuse de 1,5 à 3 nM.

*In vitro,* il suffit d'ajouter ledit polypeptide chimérique au milieu de culture des neurones. *In vivo,* il peut être administré par différentes voies, localement, notamment par injection ou infusion dans l'humeur vitrée, ou dans l'espace sous-orbital, ou sous forme de collyre, ou de pommade ophtalmique. Il peut également être administré à l'aide d'un dispositif de libération contrôlée, par exemple sous forme d'implant intra-oculaire. Le cas échéant, il peut être administré de manière systémique, par exemple par injection intraveineuse.

Les doses de polypeptide chimérique à administrer *in vivo* pour obtenir la concentration souhaitée au contact des cellules cibles, peuvent aisément être déterminées et adaptées par l'homme de l'art en fonction notamment des modalités d'administration envisagées.

On peut également effectuer cette mise en contact en mettant les cellules cibles en présence de cellules transformées pour exprimer ou surexprimer, et sécréter ledit polypeptide chimérique. *In vitro,* ceci peut s'effectuer par co-culture de ces cellules transformées avec les cellules. *In vivo,* on peut par exemple greffer dans la rétine des cellules transformées pour exprimer ou surexprimer, et sécréter ledit polypeptide chimérique.

On peut également, le cas échéant, associer ledit polypeptide chimérique avec un ou plusieurs autres principes actifs thérapeutiques, en administration conjointe ou séparée.

Des polypeptides d'adressage cellulaire conformes à l'invention peuvent également être utilisés pour inhiber la fixation d'Otx2 à ses cellules-cibles, et notamment aux neurones à parvalbumine enrobés dans un réseau périneuronal de protéoglycannes à chondroïtine sulfate, pour permettre de restaurer leur plasticité. Ils peuvent ainsi être utilisés pour le traitement de maladies résultant d'un développement défectueux au cours de la période critique de plasticité, d'une région du cerveau contenant les cellules-cibles concernées. A titre d'exemple, ils peuvent être utilisés dans le cadre du traitement de l'amblyopie, ou dans le cadre du traitement de maladies neurologiques et psychiatriques telles que les troubles anxieux, le syndrome de stress post-traumatique, ainsi que la psychose maniaco-dépressive ou la schizophrénie. Ils peuvent également être utilisés dans le cadre de la restauration de la plasticité physiologique et morphologique dans des pathologies ou des accidents vasculaires qui amènent à la perte de neurones.

Pour inhiber la fixation d'Otx2 à ses cellules-cibles, le polypeptide d'adressage cellulaire conforme à l'invention sera utilisé de manière à obtenir, au contact desdites cellules-cibles, une concentration dudit polypeptide au moins 10 fois supérieure, de préférence 100 à 1000 fois supérieure à la concentration d'Otx2. Typiquement, ledit polypeptide pourra être utilisé à une concentration de 1 à 10 $\mu$M, avantageusement de 10 à 100 $\mu$M, et de manière particulièrement avantageuse de 100 à 1000$\mu$M.

Pour l'utilisation *in vivo* afin d'inhiber la fixation d'Otx2 à ses cellules-cibles, ledit polypeptide d'adressage sera administré de préférence localement, par exemple par minipompe osmotique implantée dans le cerveau.

Les doses de polypeptide chimérique à administrer *in vivo* pour obtenir la concentration souhaitée au contact des cellules cibles, peuvent aisément être déterminées et adaptées par l'homme de l'art en fonction notamment des modalités d'administration envisagées.

Des polypeptides d'adressage cellulaire conformes à l'invention peuvent aussi être utilisés pour cribler d'autres molécules capables de se fixer spécifiquement aux cellules-cibles d'Otx2.

Dans ce cadre, la présente invention a pour objet un procédé de criblage de molécules capables de se fixer spécifiquement à des cellules-cibles d'Otx2, aux mêmes sites de fixation qu'Otx2, caractérisé en ce qu'il comprend

- la mise en présence d'un polypeptide d'adressage cellulaire conforme à l'invention, de cellules-cible d'Otx2 et de cellules ne fixant pas Otx2, et de chaque molécule à tester ;
- la sélection des molécules capables d'inhiber la fixation dudit polypeptide d'adressage cellulaire aux cellules-cible d'Otx2, et ne se fixant pas aux cellules ne fixant pas Otx2.

De manière avantageuse, ledit procédé est mis en oeuvre en présence à la fois de cellules-cibles d' Otx2, et de cellules ne fixant pas Otx2, par exemple sur une coupe de rétine.

La présente invention sera mieux comprise à l'aide du complément de description qui va suivre, qui se réfère à des exemples non-limitatifs illustrant l'identification d'un polypeptide d'adressage conforme à l'invention et la mise en évidence de sa spécificité d'adressage.

## EXEMPLE 1. IDENTIFICATION D'UNE SEQUENCE QUI ADRESSE OTX2 AUX NEURONES GANGLIONNAIRES ET BIPOLAIRES RETINIENS.

Au cours d'expérimentations précédentes (cf. Demande PCT/FR 2009/000031 du 9 janvier 2009) il a été remarqué qu'Otx2 injecté dans l'oeil se concentrait essentiellement dans les neurones ganglionnaires rétiniens (RCGs).

Afin de rechercher si un domaine d'adressage cellulaire était présent dans la séquence d'Otx2, des protéines chimériques comprenant un domaine phosphatase alcaline couplé à la protéine Otx2 entière ou à différents fragments de cette protéine ont été construites.

Les protéines de fusion suivantes ont été construites :

- Phosphatase Alcaline-Otx2 entière (AP-Otx2)
- Phosphatase Alcaline-région C-terminale+ homéodomaine d'Otx2 (AP-Ct-Otx2)
- Phosphatase Alcaline-région N-terminale+ homéodomaine d'Otx2 (AP-Nt-Otx2)
- Phosphatase Alcaline-homéodomaine d'Otx2 (AP-Hd-Otx2)

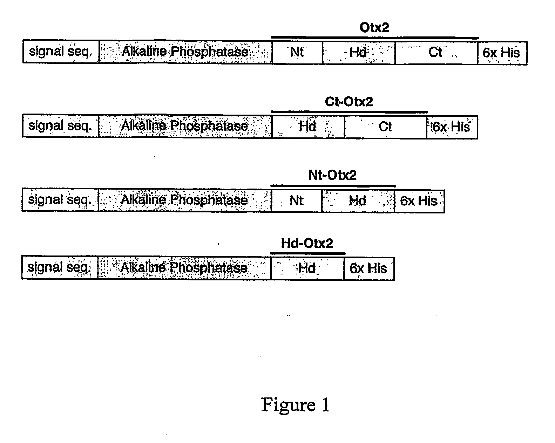

Les séquences codant pour la protéine Otx2 humaine ou pour les fragments testés ont été clonées dans le vecteur pAPtag-5 (GenHunter), en phase avec la séquence codant pour la phosphatase alcaline. Les différentes constructions réalisées sont schématisées sur la Figure 1.

Légende de la Figure 1 : signal seq. : peptide signal de la phosphatase alcaline ; Alkaline Phosphatase : phosphatase alcaline, Nt : région N-terminale d'Otx2 (acides aminés 1-37) ; Hd : homéodomaine d'Otx2 (acides aminés 38-97) ; Ct : région C-terminale d'Otx2 (acides aminés 98-289) ; 6xHis : étiquette poly-histidine.

Des cellules HEK 293 cultivées dans des boîtes de culture de 10 cm de diamètre ont été transfectées avec 10 μg de chacun des vecteurs construits, préalablement purifié à l'aide de la Lipofectamine 2000 (Invitrogen) en suivant les instructions du fabricant. Les cellules transfectées ont été incubées pendant 48 heures dans du DMEM/F12, complété avec 10% de sérum bovin foetal (SVF). Le surnageant a été collecté, centrifugé à 100 x g pendant 5 minutes et conservé à - 80 °C. Ce surnageant est utilisé sans purification pour tester la fixation des protéines chimères sur des sections de rétine.

Pour effectuer le test de fixation, des sections au cryostat (20 μm) de rétines fraîches congelées sont fixées pendant 8 minutes dans 100% de méthanol refroidi, puis lavées 3 fois 10 dans du tampon phosphate (PBS) avec 4mM de MgCl2.

Les sections sont incubées dans du tampon PBS, 4 mM MgCl2, plus 10 % SVF pendant 1 heure à température ambiante (RT).

Pour la fixation, les surnageants contenant les protéines de fusion testées sont dilués au vingtième dans du PBS et incubés pendant 2 heures à température ambiante.

Les sections sont alors lavées 5 fois en PBS, 4 mM MgCl2, et les ligands liés sont alors fixés pendant 2 minutes (60 % d'acétone, 4 % PFA, 20 mM Hepes pH 7).

Après 3 lavages en PBS, les sections sont chauffées à 65 °C pendant 2 heures en PBS pour inactiver les phosphatases endogènes. Les sections sont alors lavées 2 fois en PBS avant révélation de l'activité phosphatase alcaline (pré-incubation de 5 minutes des sections dans 100 mM Tris pH 9.5, 100 mM NaCl, 5 mM MgCl2, suivie par l'addition de NBT/BCIP (Promega)).

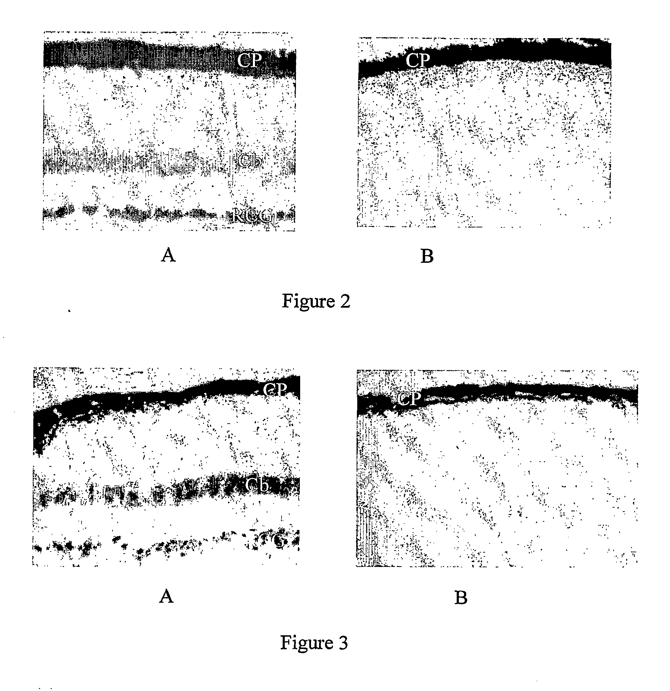

Les résultats obtenus avec les protéines de fusion AP-Nt-Otx2 et AP-Hd-Otx2.sont illustrés par la Figure 2

Légende de la Figure 2 :

A : Marquage obtenu avec la protéine de fusion AP-Nt-Otx2.
B : Marquage obtenu avec la protéine de fusion AP-Hd-Otx2.
CP : cellules de l'épithélium pigmentaire rétinien ; Cb : cellules bipolaires ; RCG : cellules ganglionnaires.

Ces résultats montrent que la protéine de fusion comprenant le domaine N-terminal d'Otx2 et son homéodomaine se fixe spécifiquement sur les RGCs et les cellules bipolaires, alors que la protéine de fusion contenant seulement l'homéodomaine ne se fixe à aucune des cellules rétiniennes.

Des peptides biotinylés correspondant à différents fragments de la portion domaine N-terminal + homéodomaine d'Otx2 ont été synthétisés et leur fixation sur des sections de rétines, préparées comme décrit ci-dessus, a été testée. L'incubation des peptides avec les sections de rétine a été effectuée dans les mêmes conditions que celles décrites ci-dessus, puis les sections portant les peptides fixés ont été incubées avec de la streptavidine marquée à la phosphatase alcaline, et l'activité phosphatase alcaline a été détectée comme décrit ci-dessus.

L'un des peptides testés (RK-Otx2), correspondant à la séquence RKQRRERTTFTRAQL (SEQ ID NO:2) possède la même spécificité de fixation que les protéines de fusion AP-Otx2 et AP-Nt-Otx2.

Des mutations ont alors été effectuées dans ce polypeptide. L'un des mutants (AA-Otx2), dans lequel deux acides aminés basiques (RK) sont changés pour deux acides aminés neutres (AA) et qui répond donc à la séquence AAQRRERTTFTRAQL SEQ ID NO: 3) ne présente aucune fixation aux cellules rétiniennes.

Ces résultats sont illustrés par la Figure 3.

Légende de la Figure 3 :

A : Marquage obtenu avec le peptide RK-Otx2.
B : Marquage obtenu avec le peptide AA-Otx2.
CP : cellules de l'épithélium pigmentaire rétinien ; Cb : cellules bipolaires ; RCG : cellules ganglionnaires.

Ces résultats montrent que le peptide RK-Otx2 se fixe, comme AP-Nt-Otx2, sur les cellules ganglionnaires et bipolaires. En revanche, aucune fixation n'est observée avec le peptide AA-Otx2. Un autre peptide (RA-Otx2), dans lequel le dipeptide RK est substitué par RA ne se fixe que très faiblement sur les cellules ganglionnaires et bipolaires (résultats non montrés).

Afin de vérifier que le peptide RK-Otx2 possédait bien la même spécificité de fixation qu'AP-Nt-Otx2, la capacité des polypeptides RK-Otx2 et AA-Otx2 à antagoniser la fixation d'AP-Nt-Otx2 a été testée. Le test de fixation d'AP-Nt-Otx2 sur des sections de rétines a été effectué comme décrit ci-dessus, à ceci près que l'incubation du surnageant contenant AP-Nt-Otx2 a été effectuée en présence de 2 μg/ml du peptide RK-Otx2 ou du peptide AA-Otx2).

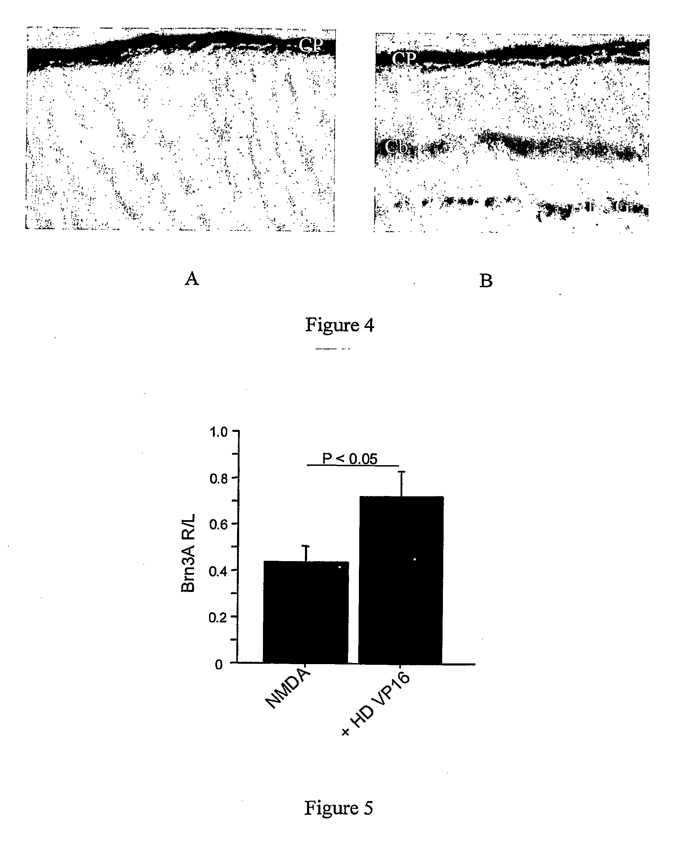

Les résultats sont illustrés par la Figure 4.

Légende de la Figure 4 :

A : Fixation d'AP-Nt-Otx2 en présence du peptide RK-Otx2.

B : Fixation d'AP-Nt-Otx2 en présence du peptide AA-Otx2.

CP : cellules de l'épithélium pigmentaire rétinien ; Cb : cellules bipolaires ; RCG : cellules ganglionnaires.

Ces résultats montrent que le peptide RK-Otx2 bloque la fixation d'AP-Nt-Otx2 aux cellules bipolaires et aux cellules ganglionnaires, alors que le peptide AA-Otx2 n'a aucun effet sur cette fixation.

**EXEMPLE 2 : EFFET D'UN POLYPEPTIDE CHIMERIQUE ASSOCIANT UN FRAGMENT D'OTX2 CONTENANT LA SEQUENCE D'ADRESSAGE CELLULAIRE AVEC UN DOMAINE HETEROLOGUE D'ACTIVATION DE LA TRANSCRIPTION, SUR LA SURVIE DES NEURONES GANGLIONNAIRES RETINIENS.**

Il a été montré précédemment (cf. Demande PCT/FR 2009/000031 du 9 janvier 2009) qu'Otx2 protégeait les neurones ganglionnaires rétiniens contre les effets toxiques du N-methyl-D-aspartate (NMDA).

Un polypeptide chimérique a été construit génétiquement et produit par synthèse bactérienne, en fusionnant le domaine N-terminal d'Otx2 et son homéodomaine (acides aminés 1-97 d'Otx2), avec le domaine trans-activateur VP16 du virus de l'herpès (MLGDGDSPGPGFTPHDSAPYGALDMADFEFEQMFTDALGIDEYGG, SEQ ID NO: 4).

Des souris C57 B16 ont reçu dans l'oeil droit, 1 μl de tampon d'injection (PBS ou NaCl 9 ‰) contenant soit 1mM de NMDA, soit 1mM de NMDA additionné de 30 ng du polypeptide chimérique, et dans l'oeil gauche, le même volume de tampon d'injection, sans additif.

La survie des neurones ganglionnaires a été déterminée en mesurant le niveau d'expression de Brain 3A (Brn3A), un facteur de transcription qui dans la rétine, est spécifiquement exprimé dans les neurones ganglionnaires (XIANG et al., J. Neurosci., 15, 4762-4785, 1995).

Au bout de 4 jours, les animaux sont sacrifiés, les rétines prélevées, et l'ARNm en est extrait.

Le niveau d'expression de l'ARNm de Brn3A a été déterminé par RT-PCR quantitative, en utilisant le gène de l'hypoxanthine phosphoribosyltransférase (HPRT) comme gène de référence, et le rapport entre l'expression de l'ARNm de Brn3A dans l'oeil droit et dans l'oeil gauche a été calculé.

Les résultats sont illustrés par la Figure 5. En abscisse, sont indiqués les additifs utilisés ; en ordonnée, est indiqué le rapport entre les quantités d'ARNm Brn3A (normalisées par rapport à l'ARNm HPRT) dans l'oeil droit et dans l'oeil gauche.

Ces résultats montrent que le NMDA, administré seul, diminue significativement (d'environ 60%) la quantité des neurones ganglionnaires, et que l'addition de 30 ng du polypeptide chimérique protège efficacement les neurones ganglionnaires contre les effets toxiques du NMDA.

**EXEMPLE 3: LIAISON D'OTX2 A DES CELLULES CIBLES DU CORTEX CEREBRAL.**

L'interaction avec les cellules du cortex cérébral des protéines de fusion AP-Otx2, AP-Nt-Otx2, AP-Hd-Otx2, et AP-HdAA-Otx2 (variant de AP-Nt-Otx2 dans lequel le doublet d'acides aminés RK a été remplacé par le doublet AA), seules (surnageant de culture dilué au 1/20ème) ou en présence d'Otx2 entière (1 ug/ml) ou des peptides RK-Otx2 ou AA-Otx2 (2 μg/ml), a été testée sur des sections au cryostat de cerveaux de souris adultes, en utilisant le protocole décrit dans l'Exemple 1 ci-dessus.

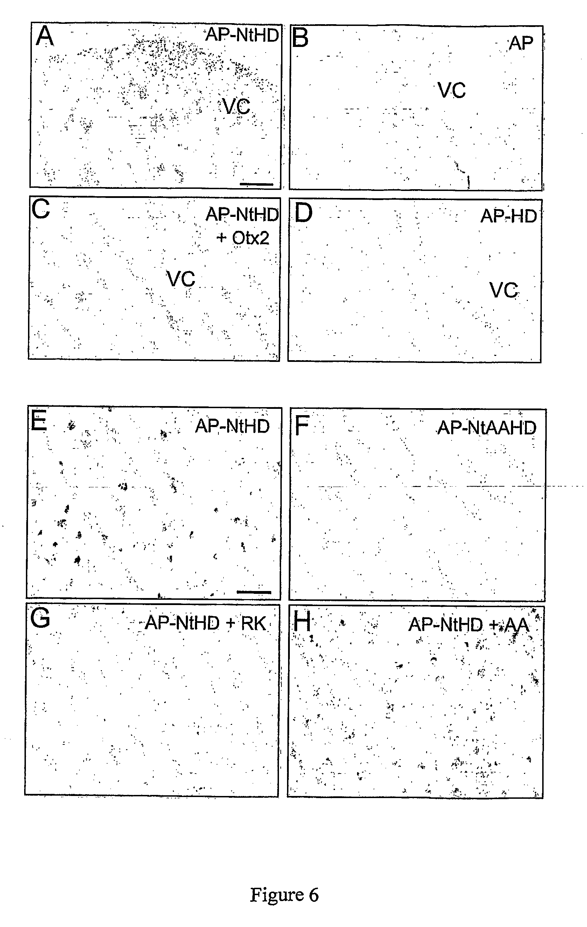

Les résultats sont illustrés par la Figure 6.

Légende de la figure 6: A-D, barre d'échelle 500μm ;(A) AP-Nt-Otx2; (B) AP (phosphatase alcaline seule) ; (C) AP-Nt-Otx2 en présence d'Otx2 entière; (D) AP-Hd-Otx2; E-H, barre d'échelle 100μm ; (E) AP-Nt-Otx2; (F) AP-HdAA-Otx2 ; (G) AP-Nt-Otx2 en présence de RK-Otx2 (H) AP-Nt-Otx2 en présence d'AA-Otx2.

Ces résultats montrent qu'AP-Nt-Otx2 se lie à des cellules corticales, comprenant celles du cortex visuel ; en revanche, aucune liaison n'est observée avec AP, AP-Hd-Otx2, ou AP-HdAA-Otx2. En outre, Otx2 entière ainsi que RK-Otx2, mais pas AA-Otx2 bloquent la liaison d'AP-Nt-Otx2 à ses cellules cibles.

Les glycosaminoglycannes (GAGs) et notamment les protéoglycannes à chondroitine sulfate, sont un constituant essentiel de la matrice extra-cellulaire (réseau périneuronal) qui enrobe les neurones à parvalbumine du cortex visuel. La mise en place de ce réseau périneuronal coïncide avec la fin de la période critique de plasticité ; il constitue un facteur majeur de la perte de plasticité corticale survenant à l'issue de cette période critique, et il a été montré que la destruction de ce réseau périneuronal par traitement à la chondroïtinase-ABC permettait de restaurer cette plasticité (PIZZORUSSO et al., Science, 298, 1248-51, 2002).

Pour déterminer le réseau périneuronal associé aux neurones à parvalbumine était impliqué dans la liaison entre AP-Nt-Otx2 et ses cellules cibles, la fixation d'AP-Otx2 a été testée sur des sections au cryostat de cerveaux de souris adultes fixées au méthanol, puis incubées pendant 24 heures en présence de Chondroïtinase ABC (2U/ml) dans un tampon contenant 50mM Tris [pH 8,0], 40mM d'acétate sodium, 0,1 % BSA et des inhibiteurs de protéases. Parallèlement, les coupes, traitées ou non à la Chondroïtinase ABC, ont été incubées avec 0,01 mg/ml de lectine *Wisteria*

*floribunda agglutinin* (WFA; Sigma-Aldrich), qui se fixe aux GAGs du réseau périneuronal, marquée à la FITC.

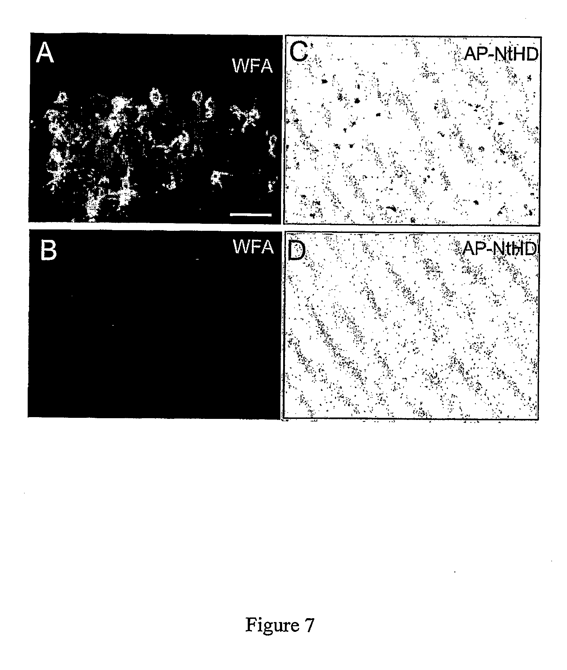

Les résultats sont illustrés par la Figure 7.

Légende de la Figure 7 : (A) et (B) marquage à la WFA ; (C) et (D) incubation en présence d'AP-Otx2 ;(A) et (C) coupes non-traitées ; (B) et (D) coupes traitées à la Chondroïtinase ABC.

Ces résultats montrent que le traitement à la Chondroïtinase ABC, qui détruit les GAGs du réseau périneuronal abolit également la fixation d'AP-Nt-Otx2.

Il apparaît donc que ce sont les GAGs du réseau périneuronal associé aux neurones à parvalbumine qui portent le site de fixation d'AP-Nt-Otx2.

## EXEMPLE 4: BLOCAGE *IN VIVO* DU TRANSFERT ENDOGENE D'OTX2 PAR LE PEPTIDE RK-OTX2.

Comme montré ci-dessus, le peptide RK-Otx2 peut bloquer *in vitro* la liaison d'Otx2 à ses cellules cibles. Il a été testé si cet effet se produisait *également in vivo.*

Dans ce but, le peptide RK-Otx2 (0,25 mg/ml), le peptide AA-Otx2 (0,25 mg/ml), ou du tampon PBS, associés à de l'acide polysialique (0,25 mg/ml, pour permettre la diffusion des peptides et éviter leur fixation non-spécifique sur des neurones exprimant à leur surface l'acide polysialique) ont été infusés lentement(1 $\mu$l/hr) pendant 7 jours dans le cortex visuel droit de souris adultes, à l'aide de minipompes osmotiques (Alzet 1003D, Alza) connectées à des canules implantées stéréotaxiquement (HENSCH et al., Science, 282, 1504-8, 1998; FAGIOLINI & HENSCH, Nature, 404, 183-6, 2000). A la fin de l'infusion, les souris sont perfusées avec 4% de PFA, et des coupes de cerveau (25$\mu$m) sont effectuées pour étudier la la localisation d'Otx2 et celle du peptide RK-Otx2. Otx2 est visualisé à l'aide d'un anticorps monoclonal de rat anti-Otx2 dilué au 1/200$^{ème}$ (SUGIYAMA et al., Cell, 134, 508-20, 2008), suivi d'un anticorps d'âne anti-rat marqué à l'Alexa 488 (Molecular Probes) dilué au 1/2000$^{ème}$. Le peptide RK-Otx2 est visualisé à l'aide de streptavidine marquée au Cy5.

Les cellules exprimant Otx2 ont été comptées sur une surface de 700x350 $\mu$m englobant les couches II/III et IV de la zone binoculaire du cortex visuel.

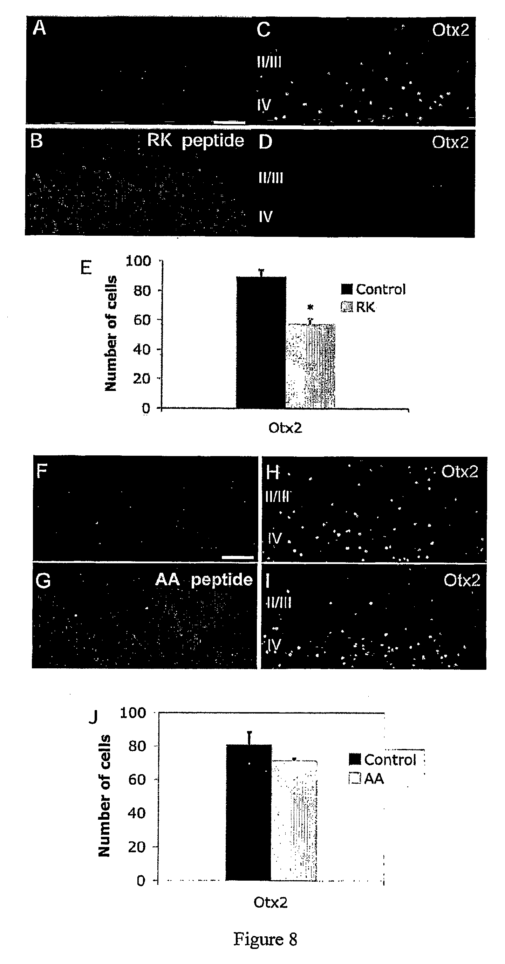

Les résultats sont illustrés par la Figure 8.

Légende de la Figure 8 :

A-E : infusion de RK-Otx2 (barre d'échelle =100$\mu$m)

A, B : détection du peptide RK-Otx2 ; A : hémisphère contrôle ; B : hémisphère infusé.

C, D : Détection d'Otx2; C : hémisphère contrôle ; D : hémisphère infusé.

E: Quantification des cellules exprimant Otx2 ; barres noires : hémisphère contrôle ; barres grises : hémisphère infusé.

F-J : infusion d'AA-Otx2 (barre d'échelle = 100$\mu$m)

F, G : détection du peptide AA-Otx2 ; A : hémisphère contrôle ; B : hémisphère infusé.

H, I : Détection d'Otx2; H : hémisphère contrôle ; I : hémisphère infusé.

J: Quantification des cellules exprimant Otx2 ; barres noires : hémisphère contrôle ; barres grises : hémisphère infusé ; * p <0.005, Test t de student par séries appariées ; les barres d'erreur représentent l'erreur type de la moyenne..

Ces résultats montrent que l'infusion du peptide RK-Otx2 dans le cortex visuel pendant 7 jours réduit significativement le nombre de cellules exprimant Otx2. en revanche, dans le cas du peptide AA-Otx2, on n'observe qu'une faible réduction, non significative, du nombre de cellules exprimant Otx2.

Il a été vérifié, par marquage au Sytox green, que l'infusion n'avait pas d'effet en elle-même sur le nombre de cellules. Pour s'assurer que la diminution du nombre de cellules exprimant Otx2 n'était pas due à la mort des cellules, une infusion de ce peptide a été effectuée selon le protocole décrit ci-dessus, et le nombre de cellules exprimant Otx2 a été mesuré 8 jours après la fin de l'infusion. Dans ces conditions, on ne détecte plus, 8 jours après la fin de l'infusion, que de très faibles quantités de peptideRK-Otx2, et le nombre de cellules exprimant Otx2 dans l'hémisphère traité est restauré au niveau de celui de l'hémisphère témoin.

## EXEMPLE 5: RESTAURATION DE LA PLASTICITE CORTICALE PAR LE PEPTIDE RK-OTX2

Les effets du peptide RK-Otx2 sur la plasticité du cortex oculaire ont été comparés avec ceux de la chondroïtinase-ABC, connue pour permettre la restauration de cette plasticité (PIZZORUSSO et al., Science, 298, 1248-51, 2002).

Le peptide RK-Otx2 ou le peptide AA-Otx2 ont été infusés chez des souris adultes (donc après la fermeture de la période critique de plasticité), comme décrit à l'Exemple 4 ci-dessus. La chondroïtinase-ABC, ou le tampon d'injection (eau désionisée + 0.1% SAB) ont été injectés (0,4 $\mu$l pour chaque injection) en 3 sites entourant le cortex visuel (AP lambda, LM 1.5mm; AP lambda, 4.0mm ; AP + 1.5 mm ; LM 2.5 mm) à deux profondeurs différentes (300 et 500 $\mu$m).

Après l'infusion des peptides ou l'injection de chondroïtinase-ABC, les souris sont soumises à une privation monoculaire pendant 4 jours, et les réponses aux stimuli visuels sont ensuite mesurées par électrophysiologie unitaire extracellulaire. Les enregistrements électrophysiologiques sont effectués sous anesthésie nembutal/chlorprothixene anesthesia using standard techniques (GORDON & STRYKER, J Neurosci, 16, 3274-86, 1996; MATAGA et al., Neuron, 44, 1031-41, 2004). 5 à 7 enregistrements unitaires ont été effectués pour chaque souris, des deux cotés de l'axe médio-latéral du cortex visuel primaire, pour couvrir la zone monoculaire et la zone binoculaire, et éviter des biais d'échantillonnage. Des scores de dominance cellulaire ont été attribués aux réponses cellulaires, en utilisant un système de classification en 7 points (WIESEL & HUBEL, J Neurophysiol, 26, 978-93, 1963) (GORDON & STRYKER, J Neurosci, 16, 3274-86, 1996). La dominance oculaire dans la zone binoculaire a été calculée pour chaque souris selon un index de biais controlatéral(CBI), déterminé comme suit:

$$(CBI) : [(n_1-n_7+2/3(n_2-n_6) + 1/3(n_3-n_5)+N]/2N, \text{ où } N=\text{nombre total de}$$

où N=nombre total de cellules et nx=nombre de cellules correspondent à un score de dominance oculaire de x.

Cette moyenne pondérée du biais en faveur de l'un ou l'autre oeil peut varier entre 0, pour une dominance ipsilatérale complète et 1 pour une dominance controlatérale complète.

En outre, des coupes frontales des cerveaux des souris traitées ont été effectuées, comme décrit à l'Exemple 4 ci-dessus, afin de déterminer l'influence du peptide RK-Otx2 sur les neurones à parvalbumine. Les coupes ont été maquées soit à la WFA, comme décrit à l'Exemple 3, soit à l'aide d'un anticorps monoclonal de souris anti-parvalbumine (1/500, Sigma-Aldrich), qui a été révélé à l'aide d'in anticorps d'âne anti-souris, marqué au Cy3. Les cellules marquées ont été quantifiées comme décrit à l'Exemple 4.

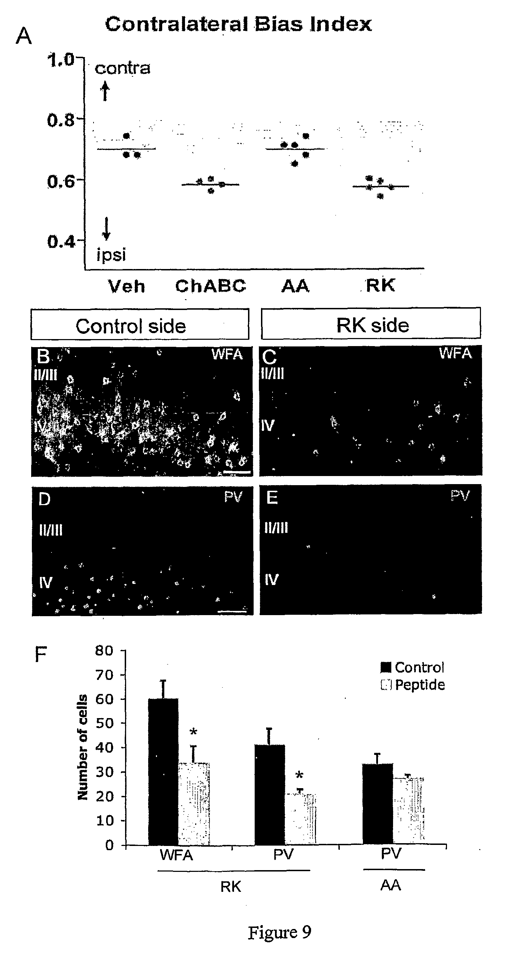

Les résultats sont illustrés par la Figure 9.

Légende de la Figure 9 :

A : indice de biais controlatéral après infusion du peptide RK-Otx2 (RK) ou du peptide AA-Otx2 (AA), ou après injection de chondroïtinase-ABC (chABC) ou de tampon d'injection seul(Veh)

B-E : marquage à la WFA (B et C)et expression de la parvalbumine (D et E) dans la région supragranulaire du cortex visuel de l'hémisphère infusé avec le peptide RK (C et E), et de l'hémisphère non infusé (barre d'échelle =100μm).

F: Quantification des cellules marquées à la WFA et des cellules exprimant la parvalbumine après infusion du peptide RK-Otx2 (RK) ou du peptide AA-Otx2 (AA) ; barres noires : hémisphère contrôle ; barres grises : hémisphère infusé ; * p <0.05, Test t de student par séries appariées ; les barres d'erreur représentent l'erreur type de la moyenne.

Ces résultats montrent que la privation monoculaire induit une dominance oculaire (abaissement de l'indice de biais contralatéral de 0,7 à 0,57) chez les souris adultes traitées par le peptide RK-Otx2, comme chez celles traitées par la chondroïtinase-ABC (chABC), à la différence des souris traitées avec le tampon d'injection ou le peptide AA-Otx2.

Parallèlement, l'infusion du peptide RK-Otx2, (mais pas celle du peptide AA-Otx2) diminue l'expression des sites de liaison de la WFA, ainsi que celle de la parvalbumine. Le nombre de cellules positives pour la parvalbumine diminue de 56.2 %, et celui de cellules entourées par des sites de liaison du WFA diminue de 51.3 %.

Il ressort de ces résultats que le blocage du transfert d'Otx2 par le peptide RK-Otx2 provoque l'inhibition de l'expression de la parvalbumine, ainsi qu'une destruction du réseau périneuronal similaire à celle provoquée par la chondroïtinase-ABC. Il en résulte un retour des neurones à parvalbumine à un état immature, similaire à celui observé habituellement pendant la période critique, et cet état immature permet la réouverture de la période critique, et la restauration de la plasticité qui lui est associée.

## Revendications

1. Polypeptide d'adressage cellulaire, **caractérisé en ce qu'**il est défini par la séquence $B_1B_2X_1B_3B_4X_2B_5X_3X_4X_5X_6B_6X_7X_8X_9$ (SEQ ID NO: 1), dans laquelle :

   $B_1$, $B_2$, $B_3$, $B_4$, $B_5$ et $B_6$ représentent indépendamment l'arginine ou la lysine ;
   $X_1$ et $X_8$ représentent indépendamment l'asparagine ou la glutamine ;
   $X_2$ représente l'acide aspartique ou l'acide glutamique ;
   $X_3$, $X_4$ et $X_6$ représentent indépendamment la thréonine ou la sérine ;
   $X_5$ représente la phénylalanine, la tyrosine ou le tryptophane ;
   $X_7$ représente l'alanine ou la glycine ;
   $X_9$ représente la leucine, l'isoleucine ou la valine,
   et **en ce que** lorsqu'il est mis en présence de cellules rétiniennes, il se lie spécifiquement aux neurones ganglionnaires et aux neurones bipolaires.

**2.** Polypeptide d'adressage cellulaire selon la revendication 1, **caractérisé en ce que** :

- au moins un des acides aminés $B_1$, $B_3$, $B_4$, $B_5$ et $B_6$ est une arginine ; et/ou
- $B_2$ est une lysine ; et/ou
- au moins un des acides aminés $X_1$ et $X_8$ est une glutamine ; et/ou
- $X_2$ est un acide glutamique ; et/ou
- au moins un des acides aminés $X_3$ $X_4$ et $X_6$ est une thréonine ; et/ou
- $X_5$ est une phénylalanine ; et/ou
- $X_7$ est une alanine ; et/ou
- $X_9$ est une leucine.

**3.** Polypeptide d'adressage cellulaire selon la revendication 2, **caractérisé en ce qu'**il est défini par la séquence suivante : RKQRRERTTFTRAQL (SEQ ID NO: 2).

**4.** Polypeptide contenant un polypeptide d'adressage cellulaire selon une quelconque des revendications 1 à 3 et un polypeptide transducteur.

**5.** Composition comprenant un polypeptide selon une quelconque des revendications 1 à 4, lié à un cargo d'intérêt.

**6.** Composition selon la revendication 5, **caractérisée en ce qu'**elle est constituée par un polypeptide chimérique contenant un polypeptide selon une quelconque des revendications 1 à 4 et un ou plusieurs polypeptides constituant le cargo.

**7.** Composition selon la revendication 6, **caractérisée en ce que** ledit polypeptide chimérique contient :

- un polypeptide selon la revendication 4, et
- un cargo comprenant une ou plusieurs séquences de régulation de la transcription et/ou une ou plusieurs séquences de régulation de la traduction.

**8.** Utilisation d'un polypeptide selon une quelconque des revendications 1 à 3, pour l'adressage spécifique *in vitro* d'un cargo d'intérêt à des cellules-cibles d'Otx2.

**9.** Polypeptide selon une quelconque des revendications 1 à 3, pour une utilisation diagnostique ou thérapeutique permettant l'adressage spécifique d'un cargo d'intérêt à des cellules cibles d'Otx2.

**10.** Utilisation d'un polypeptide selon la revendication 4 pour l'adressage spécifique *in vitro* d'un cargo d'intérêt à des cellules-cibles d'Otx2 et l'internalisation dudit cargo dans lesdites cellules.

**11.** Polypeptide selon la revendication 4 pour une utilisation diagnostique ou thérapeutique permettant l'adressage spécifique d'un cargo d'intérêt à des cellules cibles d'Otx2 et l'internationalisation dudit cargo dans lesdites cellules.

**12.** Utilisation d'une composition selon la revendication 7 pour augmenter in vitro la survie de cellules cibles d'Otx2.

**13.** Composition selon la revendication 7 pour l'utilisation comme médicament pour prévenir ou traiter la dégénérescence de cellules cibles d'Otx2.

**14.** Utilisation selon une quelconque des revendications 8, 10, ou 12, **caractérisée en ce que** lesdites cellules cibles d'Otx2 sont des neurones ganglionnaires ou des neurones bipolaires rétiniens.

**15.** Polypeptide selon une quelconque des revendications 1 à 3, pour l'utilisation comme médicament inhibant la fixation d'Otx2 aux neurones à parvalbumine, pour le traitement d'une maladie choisie parmi l'amblyopie, les troubles anxieux, le syndrome de stress post-traumatique, la psychose maniaco-dépressive et la schizophrénie.

**Patentansprüche**

**1.** Polypeptid für zelluläres Targeting, dadurch **gekenn- zeichnet** , dass es durch die Sequenz $B_1B_2X_1B_3B_4X_2B_5X_3X_4X_5X_6B_6X_7X_8X_9$ (SEQ ID NO:1) definiert wird, in der:

$B_1$, $B_2$, $B_3$, $B_4$, $B_5$ und $B_6$ unabhängig Arginin oder Lysin darstellen;

$X_1$ und $X_8$ unabhängig Asparagin oder Glutamin darstellen;

$X_2$ Asparaginsäure oder Glutaminsäure darstellt;

$X_3$, $X_4$ und $X_6$ unabhängig Threonin oder Serin darstellen;

$X_5$ Phenylalanin, Tyrosin oder Tryptophan darstellt;

$X_7$ Alanin oder Glycin darstellt;

$X_9$ Leucin, Isoleucin oder Valin darstellt,

und dass, wenn es in die Gegenwart von retinalen Zellen gebracht wird, es spezifisch an Ganglionneuronen und bipolare Neuronen bindet.

2. Polypeptid für zelluläres Targeting gemäß Anspruch 1, **dadurch gekennzeichnet , dass**:

- wenigstens eine der Aminosäuren $B_1$, $B_3$, $B_4$, $B_5$ und $B_6$ ein Arginin ist und/oder
- $B_2$ ein Lysin ist und/oder
- wenigstens eine der Aminosäuren $X_1$ und $X_8$ ein Glutamin ist und/oder
- $X_2$ eine Glutaminsäure ist und/oder
- wenigstens eine der Aminosäuren $X_3$, $X_4$ und $X_6$ ein Threonin ist und/oder
- $X_5$ ein Phenylalanin ist und/oder
- $X_7$ ein Alanin ist und/oder
- $X_9$ ein Leucin ist.

3. Polypeptid für zelluläres Targeting gemäß Anspruch 2, **dadurch gekennzeichnet , dass** es durch die folgende Sequenz definiert ist: RKQRRERTTFTRAQL (SEQ ID NO: 2).

4. Polypeptid, enthaltend ein Polypeptid für zelluläres Targeting gemäß einem der Ansprüche 1 bis 3 und ein Transducer-Polypeptid.

5. Zusammensetzung, umfassend ein Polypeptid gemäß einem der Ansprüche 1 bis 4, das an ein Cargo von Interesse gebunden ist.

6. Zusammensetzung gemäß Anspruch 5, **dadurch gekennzeichnet, dass** sie aus einem chimären Polypeptid, enthaltend ein Polypeptid gemäß einem der Ansprüche 1 bis 4 und ein Polypeptid oder mehrere Polypeptide, das/die das Cargo bildet/bilden, besteht.

7. Zusammensetzung gemäß Anspruch 6, dadurch **gekenn- zeichnet**, dass das chimäre Polypeptid enthält:

- ein Polypeptid gemäß Anspruch 4 und
- ein Cargo, das eine oder mehrere Regulationssequenz(en) der Transkription und/oder eine oder mehrere Regulationssequenz(en) der Translation umfasst.

8. Verwendung eines Polypeptids gemäß einem der Ansprüche 1 bis 3 für spezifisches *In-vitro*-Targeting eines Cargo von Interesse zu Otx2-Zielzellen.

9. Polypeptid gemäß einem der Ansprüche 1 bis 3 für eine diagnostische oder therapeutische Verwendung, das das spezifische Targeting eines Cargos von Interesse an Otx2-Zielzellen erlaubt.

10. Verwendung eines Polypeptids gemäß Anspruch 4 für spezifisches *In-vitro*-Targeting eines Cargos von Interesse zu Otx2-Zielzellen und Internalisierung des Cargos in die Zellen.

11. Polypeptid gemäß Anspruch 4 für eine diagnostische oder therapeutische Verwendung, das das spezifische Targeting eines Cargos von Interesse zu Otx2-Zielzellen und die Internalisierung des Cargos in die Zellen erlaubt.

12. Verwendung einer Zusammensetzung gemäß Anspruch 7, um das Überleben von Otx2-Zielzellen *in vitro* zu erhöhen.

13. Zusammensetzung gemäß Anspruch 7 zur Verwendung als Medikament zur Prävention oder Behandlung der Degeneriertheit von Otx2-Zielzellen.

14. Verwendung gemäß einem der Ansprüche 8, 10 oder 12, **dadurch gekennzeichnet , dass** die Otx2-Zielzellen Ganglionneuronen oder bipolare retinale Neuronen sind.

15. Polypeptid gemäß einem der Ansprüche 1 bis 3 zur Verwendung als Medikament, das die Fixierung von Otx2 an Parvalbumin-Neuronen inhibiert, für die Behandlung einer Krankheit, ausgewählt aus Amblyopie, Angststörungen, posttraumatischem Stresssyndrom, manisch-depressiver Psychose und Schizophrenie.

**Claims**

1. Cell-targeting polypeptide, **characterised in that** it is defined by the sequence $B_1B_2X_1B_3B_4X_2B_5X_3X_4X_5X_6B_6X_7X_8X_9$ (SEQ ID NO:1), in which:

   $B_1$, $B_2$, $B_3$, $B_4$, $B_5$ and $B_6$ independently stand for arginine or lysine;
   $X_1$ and $X_8$ independently stand for asparagine or glutamine;
   $X_2$ stands for aspartic acid or glutamic acid;
   $X_3$, $X_4$ and $X_6$ independently stand for threonine or serine;
   $X_5$ stands for phenylalanine, tyrosine or tryptophan;
   $X_7$ stands for alanine or glycine;
   $X_9$ stands for leucine, isoleucine or valine,
   and **in that** when it is in the presence of retinal cells, it binds specifically to ganglion neurons and to bipolar neurons.

2. Cell-targeting polypeptide as claimed in claim 1, **characterised in that**:

   - at least one of the amino acids $B_1$, $B_3$, $B_4$, $B_5$ and $B_6$ is an arginine; and/or
   - $B_2$ is a lysine; and/or
   - at least one of the amino acids $X_1$ and $X_8$ is a glutamine; and/or
   - $X_2$ is a glutamic acid; and/or
   - at least one of the amino acids $X_3$, $X_4$ and $X_6$ is a threonine; and/or
   - $X_5$ is a phenylalanine; and/or
   - $X_7$ is an alanine; and/or
   - $X_9$ is a leucine.

3. Cell-targeting polypeptide as claimed in claim 2, **characterised in that** it is defined by the following sequence:

   RKQRRERTTFTRAQL (SEQ ID NO:2).

4. Polypeptide containing a cell-targeting polypeptide as claimed in any one of claims 1 to 3 and a transducer polypeptide.

5. Composition comprising a polypeptide as claimed in any one of claims 1 to 4 bonded to a cargo of interest.

6. Composition as claimed in claim 5, **characterised in that** it is made up of a chimeric polypeptide containing a polypeptide as claimed in any one of claims 1 to 4 and one or more polypeptides constituting the cargo.

7. Composition as claimed in claim 6, **characterised in that** said chimeric polypeptide contains:

   - a polypeptide as claimed in claim 4, and
   - a cargo comprising one or more transcription-regulating sequences and/or one or more translation-regulating sequences.

8. Use of a polypeptide as claimed in any one of claims 1 to 3, for specifically targeting *in vitro* a cargo of interest to target cells of Otx2.

9. Polypeptide as claimed in any one of claims 1 to 3 for diagnostic or therapeutic use, enabling a cargo of interest to target cells of Otx2 to be specifically targeted.

10. Use of a polypeptide as claimed in claim 4, for specifically targeting *in vitro* a cargo of interest to target cells of Otx2 and said cargo to be internalised in said cells.

11. Polypeptide as claimed in claim 4 for diagnostic or therapeutic use, enabling a cargo of interest to target cells of Otx2 to be specifically targeted and said cargo to be internalised in said cells.

12. Use of a composition as claimed in claim 7 to increase *in vitro* the survival of target cells of Otx2.

13. Composition as claimed in claim 7 for use as a medicament to prevent or treat the degeneration of target cells of Otx2.

14. Use as claimed in any one of claims 8, 10 or 12, **characterised in that** said target cells of Otx2 are ganglion neurons or retinal bipolar neurons.

15. Polypeptide as claimed in any one of claims 1 to 3, for use as a medicament to inhibit fixation of Otx2 to parvalbumin neurons for treating a disorder selected from amblyopia, anxiety disorders, post-traumatic stress syndrome, manic-depressive psychosis and schizophrenia.

**Otx2**

| signal seq. | Alkaline Phosphatase | Nt | Hd | Ct | 6x His |

**Ct-Otx2**

| signal seq. | Alkaline Phosphatase | Hd | Ct | 6x His |

**Nt-Otx2**

| signal seq. | Alkaline Phosphatase | Nt | Hd | 6x His |

**Hd-Otx2**

| signal seq. | Alkaline Phosphatase | Hd | 6x His |

Figure 1

A                                          B

Figure 2

A                                          B

Figure 3

A                                B

Figure 4

Figure 5

Figure 6

Figure 7

Figure 8

Figure 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009106767 A
- WO 0001417 A
- WO 0029427 A
- WO 04069279 A
- FR 2009000031 W

**Littérature non-brevet citée dans la description**

- **SIMEONE et al.** *Embo J,* 1993, vol. 12, 2735-47
- **ACAMPORA et al.** *Prog Neurobiol,* 2001, vol. 64, 69-95
- **SIMEONE et al.** *Curr Opin Genet Dev,* 2002, vol. 12, 409-15
- **SUGIYAMA et al.** *Cell,* 2008, vol. 134, 508-20
- **TOPISIROVIC ; BORDEN.** *Histol. Histopathol.,* 2005, vol. 20, 1275-1284
- **XIANG et al.** *J. Neurosci.,* 1995, vol. 15, 4762-4785
- **PIZZORUSSO et al.** *Science,* 2002, vol. 298, 1248-51
- **HENSCH et al.** *Science,* 1998, vol. 282, 1504-8
- **FAGIOLINI ; HENSCH.** *Nature,* 2000, vol. 404, 183-6
- **GORDON ; STRYKER.** *J Neurosci,* 1996, vol. 16, 3274-86
- **MATAGA et al.** *Neuron,* 2004, vol. 44, 1031-41
- **WIESEL ; HUBEL.** *J Neurophysiol,* 1963, vol. 26, 978-93

SEQUENCE LISTING

<110> CENTRE NATIONAL DE LA RECHERCHE SCIENTIFIQUE
ECOLE NORMALE SUPERIEURE
PROCHIANTZ, Alain
DI NARDO, Ariel
BEURDELEY, Marine

<120> POLYPEPTIDES D'ADRESSAGE SPECIFIQUE A DES CELLULES-CIBLES D'OTX2

<130> MJP/11-F644/235WO

<160> 4

<170> PatentIn version 3.3

<210> 1
<211> 15
<212> PRT
<213> Artificial

<220>
<223> Peptide d'adressage

<220>
<221> VARIANT
<222> (1) .. (1)
<223> Lys

<220>
<221> VARIANT
<222> (2)..(2)
<223> Arg

<220>
<221> VARIANT
<222> (3)..(3)
<223> Asn

<220>
<221> VARIANT
<222> (4)..(4)
<223> Lys

<220>
<221> VARIANT
<222> (5)..(5)
<223> Lys

<220>
<221> VARIANT
<222> (6)..(6)
<223> Asp

<220>
<221> VARIANT
<222> (7)..(7)
<223> Lys

<220>
<221> VARIANT
<222> (8)..(8)
<223> Ser

<220>
<221> VARIANT
<222> (9)..(9)
<223> Ser

<220>
<221> VARIANT
<222> (10) .. (10)
<223> Tyr

<220>
<221> VARIANT
<222> (10)..(10)
<223> Trp

<220>
<221> VARIANT
<222> (11)..(11)
<223> Ser

<220>
<221> VARIANT
<222> (12) .. (12)
<223> Lys

<220>
<221> VARIANT
<222> (13)..(13)
<223> Gly

<220>
<221> VARIANT
<222> (14) .. (14)
<223> Asn

<220>
<221> VARIANT
<222> (15) .. (15)
<223> Ile

<220>
<221> VARIANT
<222> (15)..(15)
<223> Val

<400> 1

Arg Lys Gln Arg Arg Glu Arg Thr Thr Phe Thr Arg Ala Gln Leu
1               5                   10                  15

<210> 2
<211> 15
<212> PRT
<213> Artificial

<220>
<223> Peptide d'adressage

<400> 2

```
Arg Lys Gln Arg Arg Glu Arg Thr Thr Phe Thr Arg Ala Gln Leu
 1           5                   10                  15
```

<210> 3
<211> 15
<212> PRT
<213> Artificial

<220>
<223> Peptide d'adressage: mutant inactif

<400> 3

```
Ala Ala Gln Arg Arg Glu Arg Thr Thr Phe Thr Arg Ala Gln Leu
 1           5                   10                  15
```

<210> 4
<211> 45
<212> PRT
<213> Artificial

<220>
<223> Domaine transactivateur VP16

<400> 4

```
Met Leu Gly Asp Gly Asp Ser Pro Gly Pro Gly Phe Thr Pro His Asp
 1           5                   10                  15


Ser Ala Pro Tyr Gly Ala Leu Asp Met Ala Asp Phe Glu Phe Glu Gln
              20                  25                  30


Met Phe Thr Asp Ala Leu Gly Ile Asp Glu Tyr Gly Gly
          35                  40                  45
```